# EUROPEAN PATENT SPECIFICATION

(11) **EP 0 311 322 B1**
(45) Date of publication and mention of the grant of the patent: **21.07.1993**
(21) Application number: 88309155.5
(22) Date of filing: 03.10.1988
(51) Int. Cl.: A61K 31/50, A61K 31/535, A61K 31/54, A61K 31/44, C07D 213/82

(54) **Pyridine or pyridazine derivatives as cardio-protective agents and for the treatment of ischemic disease, and process for their preparation**
Pyridin- oder Pyridazinderivate als Kardioprotektiva und zur Behandlung von ischämischen Beschwerden und Herstellungsprozess
Dérivés de pyridine ou pyridazine comme agent cardioprotecteur et pour le traitement des troubles ischémiques et procédé pour leur préparation

(30) Priority: 05.10.1987 JP 251771/87; 21.04.1988 US 184195
(43) Date of publication of application: 12.04.1989
(73) Proprietor: FUJISAWA PHARMACEUTICAL CO., LTD., Osaka-shi Osaka 541 (JP)
(72) Inventor: Takaya, Takao 5-87, Suimeidai 1-chome, Hyogo 666-01 (JP); Takasugi, Hisashi 14-33, Hamaguchinishi 1-chome, Osaka 559 (JP); Esumi, Kimio 5-11-305, Sumiyoshiyamate 1-chome, Hyogo 658 (JP); Kuno, Atsushi 24-6, Shinkofudai 5-chome, Osaka 563-01 (JP); Sakai, Hiroyoshi, Uji-shi Kyoto 611 (JP); Maeda, Kazuhiro, Yamatotakada-shi, Nara 635 (JP); Sakamoto, Yoshie, Yonago-shi Tottori 683 (JP)
(74) Representative: Pennant, Pyers

(56) References cited:
- EP-A- 0 228 845
- AGRESSOLOGIE, vol. 23, no. 1, 1982, pages 15-18, Spei Editeur, Paris, FR: H. LABORIT et al.: "Action de la minaprine sur la pression artérielle et le taux de amines cérébrales de rats spontanément hypertendus (SHR)"

## Description

This invention relates to cardioprotective agents and therapeutic agents for heart disease comprising N-containing heterocyclic compounds or their salts.

Accordingly, the invention provides for the use of N-containing heterocyclic compounds of the following formula [I] or their salts: 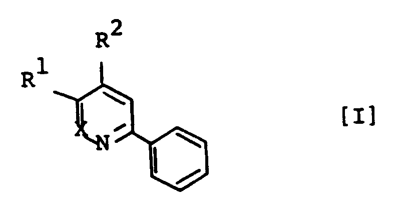
wherein
- R¹ is: C₁-C₆ alkyl substituted with C₁-C₆ alkyl substituted piperazinyl; carbamoyl substituted with morpholino(C₁-C₆)alkyl, thiomorpholino(C₁-C₆)alkyl
or C₁-C₆ alkylamino(C₁-C₆)alkyl;
piperazinylcarbonyl substituted with C₁-C₆ alkyl;
or ureido substituted with
C₁-C₆ alkylamino(C₁-C₆)alkyl; and
- R² is: phenyl substituted with nitro, and
- X is: =N- or 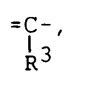 in which R³ is C₁-C₆ alkyl;
or
- R² is: C₁-C₆ alkyl, and
- X is: 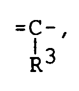 in which R³ is phenyl substituted with nitro;
for the manufacture of a medicament for the treatment of ischemic disease, depressed cardiac metabolism, reperfusion injury, myocardial infarction, heart failure or angina pectoris, in human beings and animals.

Said compounds [I] and their salts have a reducing effect on reperfusion injury and cardioprotective properties, such as improving or enhancing the depressed cardiac metabolism, and said medicaments containing them are useful in the treatment of ischemia diseases, reperfusion injury and/or the heart diseases known as myocardial infarction, heart failure and angina pectoris in human beings and animals.

It is known as described in EP-A-0228845 that the compound [I] of this invention is useful in the treatment of cerebrovascular diseases such as cerebral apoplexy [e.g. cerebral hemorrhage, cerebral infarction, and transient cerebral ischemic attack].

Particulars of the various definitions mentioned in this specification and preferred examples thereof are explained in the following.

The term "lower" used in this specification is intended to mean a group having 1 to 6 carbon atoms and preferably 1 to 4 carbon atoms, unless otherwise provided.

Suitable "C₁-C₆ alkyl" may be either a straight or branched one such as methyl, ethyl, propyl, isopropyl, butyl, tert-butyl, pentyl or hexyl, of which the most preferable is methyl.

A preferred example of "C₁-C₆ alkyl substituted with C₁-C₆ alkyl substituted piperazinyl" is methyl substituted piperazinylmethyl.

Preferred examples of "carbamoyl substituted with morpholino(C₁-C₆)alkyl" or "thiomorpholino(C₁-C₆)alkyl" include morpholinomethylcarbamoyl, morpholinoethylcarbamoyl, morpholinopropylcarbamoyl, thiomorpholinomethylcarbamoyl and thiomorpholinoethylcarbamoyl.

Suitable "C₁-C₆ alkylamino(C₁-C₆)alkyl" include mono(C₁-C₆ alkyl)amino(C₁-C₆)alkyl [e.g. methylaminomethyl, ethylaminomethyl, isopropylaminomethyl, 2-methylaminoethyl, 3-methylaminopropyl or 3-methylaminobutyl] and di(C₁-C₆ alkyl)amino(C₁-C₆)alkyl [e.g. dimethylaminomethyl, dimethylaminoethyl or 2-(N-methyl-N-ethylamino)ethyl].

Preferred examples of "carbamoyl substituted with C₁-C₆ alkylamino(C₁-C₆)alkyl" include N-(methylaminomethyl)carbamoyl, N-(dimethylaminomethyl)carbamoyl, N-(2-dimethylaminoethyl)carbamoyl, N-(2-diethylaminoethyl)carbamoyl and N-[3-(N-methyl-N-ethylamino)propyl]carbamoyl.

A preferred example of "piperazinylcarbonyl substituted with C₁-C₆ alkyl" is 4-methylpiperazin-l-ylcarbonyl.

Preferred examples of "ureido substituted with C₁-C₆ alkylamino(C₁-C₆)alkyl" include 3-(methylaminomethyl)ureido, 3-(dimethylaminomethyl)ureido, 3-(dimethylaminoethyl)ureido, 3-(diethylaminoethyl)ureido and 3-(N-methyl-N-ethylaminopropyl)ureido.

Suitable salts of the compound [I] are conventional non-toxic pharmaceutically acceptable salts and may include an organic or inorganic acid addition salt [e.g. formate, acetate, fumarate, trifluoroacetate, maleate, tartrate, methanesulfonate, benzenesulfonate, toluenesulfonate, hydrochloride, hydrobromide, sulfate or phosphate].

It is to be noted that each of the compounds of formula [I] may include one or more stereoisomers due to asymmetric carbon atom(s), and all of such isomers and a mixture thereof are included within the scope of this invention.

In order to show the usefulness of the compound [I] or their salts used as an ingredient of the cardioprotective agent and the therapeutic agent for ischemic disease, the results of pharmacological tests are shown in the following.

### Test Compounds

(a) 3-(2-Morpholinoethylcarbamoyl)-2-methyl-4-(3-nitrophenyl)-6-phenylpyridine
(b) 3-(4-Methylpiperazin-1-ylmethyl)-4-(3-nitrophenyl)-6-phenylpyridazine
(c) 4-Methyl-3-(2-morpholinoethylcarbamoyl)-2-(3-nitrophenyl)-6-phenylpyridine
(d) 2-Methyl-3-(4-methylpiperazin-1-ylcarbonyl)-4-(3-nitrophenyl)-6-phenylpyridine
(e) 3-(2-Dimethylaminoethylcarbamoyl)-2-methyl-4-(3-nitrophenyl)-6-phenylpyridine
(f) 2-Methyl-4-(3-nitrophenyl)-6-phenyl-3-[2-(4-thiomorpholinyl)ethylcarbamoyl]pyridine fumarate
(g) 3-[3-(2-Dimethylaminoethyl)ureido]-2-methyl-4-(3-nitrophenyl)-6-phenylpyridine

The compounds (a) to (e) and (g) were dissolved in 2 equivalent of hydrochloric acid and used as Test Compounds.

### Test 1

### Method :

The heart was isolated from male guinea-pig weighing 550-650 g and was perfused in the Langendorff mode at 37°C at a constant perfusion pressure of 80 cm H₂O. The perfusion medium was Krebs-Henseleit bicarbonate solution containing 11 mM glucose. The solution was equilibrated with 95% O₂ and 5% CO₂ gas mixture at pH 7.4. A latex balloon was placed in the left ventricular cavity and then left ventricular systolic (LVSP) and diastolic (LVDP) pressure were measured. Heart rate (HR) was triggered by the pulse pressure. Coronary flow was monitored with electromagnetic flow probe placed on the aorta.

The heart was perfused for 45 min. with the perfusion medium and then for 15 min. with the perfusion medium containing the test compound. The heart was then subjected to the global ischemia by stopping the perfusion. After 35 min. of ischemia, the heart was reperfused by the perfusion medium containing no test compound. At the end of 40 min. reperfusion, the cardiac function was monitored. The heart was immidiately frozen for the estimation of ATP content.

### Results :

| Test Compound | Concentration (g/ml) | % change from control | | |
|---|---|---|---|---|
| | | Cardiac depression (pre-ischemia) LVSP x HR | recovery of ATP content (% increase) | coronary flow |
| (a) | 1 x 10⁻⁶ | 4.1 | 96.3 | -1.6 |
| | 1 x 10⁻⁵ | -1.7 | 89.5 | 32.0 |
| (b) | 1 x 10⁻⁵ | -95.8 | 100.4 | -30.6 |
| (c) | 1 x 10⁻⁵ | -38.9 | 71.3 | 31.9 |
| (d) | 1 x 10⁻⁵ | -29.7 | 123.6 | 5.0 |
| (e) | 1 x 10⁻⁵ | -90.6 | 76.3 | -10.2 |
| (f) | 1 x 10⁻⁵ | -45.2 | 64.9 | 22.5 |
| (g) | 1 x 10⁻⁵ | -85.7 | 78.6 | -42.7 |

### Test 2

### Method :

Male SD rats weighing 270-340 g were anesthetized with sodium pentobarbital. The chest was opened, and then a thread was placed around the left coronary artery near the origin. Both ends of the thread were passed through a small plastic tube. After standing for 5 minutes, test compound was administered intravenously. After 10 minutes, the left coronary artery was occluded for 5 minutes by pulling the thread and pressing the plastic tube to the artery and then reperfusion was achieved by releasing the occlusion for 10 minutes. Electrocardiograms were recorded and analyzed for checking inhibitory rate of incidence of ventricular tachycardia (VT) and ventricular fibrillation (VF) in comparison with control group, and inhibitory rate of mortality as compared with control group was also calculated. 11-18 rats were used for each test group.

### Results:

| Test Compound | Dose (mg/kg) | Inhibition (%) of incidence as compared with control group | | |
|---|---|---|---|---|
| | | VT | VF | Mortality |
| (a) | 1.0 | 0 | 28.0 | 13.5 |
| | 3.2 | 0 | 60.4 | 52.5 |
| | 10 | 5.6 | 82.4 | 78.9 |

### Test 3

### Method :

Ten male SD rats weighing 140-170 g were used for each group. Test compound was administered intraperitoneally, and then myocardial infarction was induced by administering DL-isoproterenol hydrochloride (120 mg/kg) subcutaneously. After 24 hours, mortality was examined. Survival rats were anesthetized and their hearts were taken out and frozen immediately for the measurement of myocardial ATP content.

### Results :

| Test Compound | Dose (mg/kg) | Inhibition (%) of mortality * | Recovery (%) of ATP content |
|---|---|---|---|
| (a) | 1.0 | 75 | 67 |

| | | | |
|---|---|---|---|
| * % Change as compared with mortality rate in control group. | | | |

### Test 4

### Method :

Five male ddY mice weighing 27-32 g were used for each group. After test compound was administered intravenously, the right leg was occluded by binding 10 times a rubber band (diameter : 42 mm) round it. After 20 minutes later, the rubber band was cut and thickness of foot pad was measured by a dial gage immediately and 20 minutes after cutting the band. The difference of these two values was regarded as edema.

### Results :

| Test Compound | Dose (mg/kg) | Inhibition (%) of foot edema |
|---|---|---|
| (a) | 0.1 | 12 |
| | 1.0 | 30 |
| | 10 | 42 |
| (d) | 1.0 | 41.5 |
| (f) | 1.0 | 22 |

As being apparent from the above test results, the compounds [I] and their salts reduce the reperfusion injury and maintain the ATP content against the overload on the heart. Therefore, they are useful as therapeutic agents for ischemic disease and as cardioprotective agents, especially drugs to improve or enhance the depressed cardiac metabolism, and are useful in the treatment of ischemia diseases, reperfusion injury and/or the heart diseases of myocardial infarction, heart failure, angina pectoris.

The cardioprotective agent and the therapeutic agent for ischemic disease used in the present invention can be administered orally or parenterally to a mammal including human being in a conventional pharmaceutical form such as capsules, micro-capsules, tablets, granules, powders, troches, pills, ointments, suppositories, injection solutions and syrups.

The cardioprotective agent and the therapeutic agent for ischemic disease of the present invention can be produced by the established procedures using various organic or inorganic carriers, which are conventional for pharmaceutical purpose, such as excipient [e.g. sucrose, starch, mannit, sorbit, lactose, glucose, cellulose, talc, calcium phosphate and calcium carbonate], binding agent [e.g. cellulose, methyl cellulose, hydroxymethyl cellulose, polypropylpyrrolidone, gelatin, gum arabic, polyethylene glycol, sucrose and starch], disintegrator [e.g. starch, carboxymethyl cellulose, hydroxypropyl starch, sodium bicarbonate, calcium phosphate and calcium citrate], lubricant [e.g. magnesium stearate, aerosil, talc and sodium laurylsulfate], flavoring agent [e.g. citric acid, mentol, glycine and orange powders], preservative [e.g. sodium benzoate, sodium bisulfite, methylparaben and propylparaben], stabilizer [e.g. citric acid, sodium citrate and acetic acid], suspending agent [e.g. methyl cellulose, polyvinylpyrrolidone and aluminium stearate], dispersing agent [e.g. hydroxypropylmethyl cellulose], diluting agent [e.g. water], base wax [e.g. cacao butter, white petrolatum and polyethylene glycol].

While a dosage or therapeutically effective amount of the cardioprotective agent and the therapeutic agent for ischemic disease of this invention varies according to the age and conditions of each individual patient to be treated, a daily dose of about 0.1-100 mg/kg, preferably 1 to 50 mg/kg of the active ingredient may be generally given for treating diseases.

The pharmaceutical compositions of this invention comprises, as an active ingredient, the compound [I] or its salt in an amount of about 0.2 mg to about 500 mg, per dosage unit for oral and parenteral use.

The compound [I], wherein R¹ is carbamoyl substituted with morpholino(C₁-C₆)alkyl, thiomorpholino(C₁-C₆)alkyl or with C₁-C₆ alkylamino(C₁-C₆)alkyl, or piperazinylcarbonyl substituted with C₁-C₆ alkyl, R² is phenyl substituted with nitro, and R³ is C₁-C₆ alkyl, can be prepared by reacting a compound [II] or its salt with a compound [III], then reacting the resultant mixture with a compound [IV] and ammonia or an agent which liberates ammonia, and finally, if necessary, reacting the resultant mixture with an oxidizing agent without isolating intermediate products, as illustrated in the following reaction scheme. 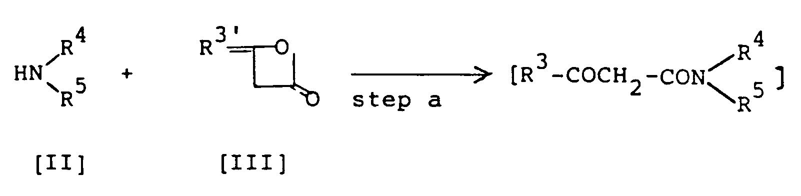
or its salt 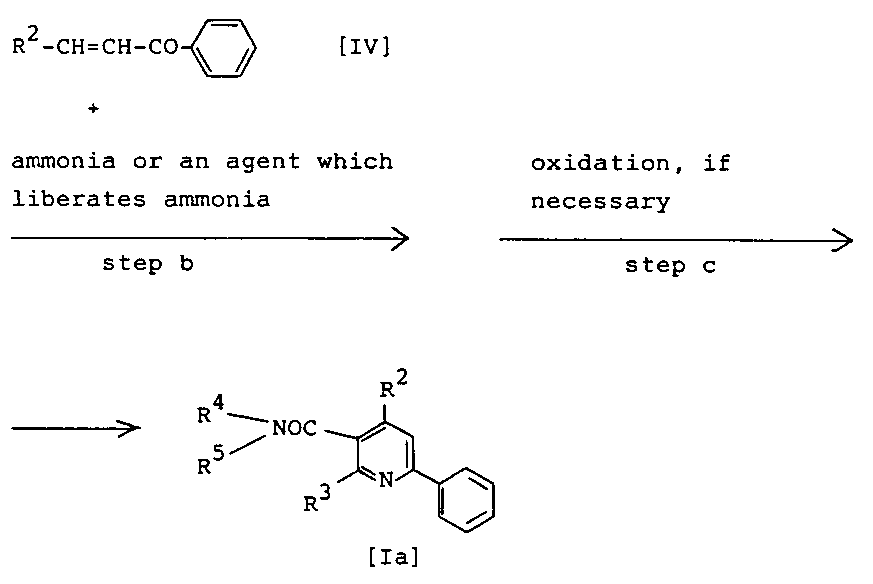
or its salt wherein
- R² is: phenyl substituted with nitro,
- R³ is: C₁-C₆ alkyl,
- R³ is: C₁-C₆ alkylidene, and
- R⁴ is: morpholino(C₁-C₆)alkyl, thiomorpholino(C₁-C₆)alkyl or C₁-C₆ alkylamino(C₁-C₆)alkyl, and
- R⁵ is: hydrogen; or
- R⁴ and R⁵: are taken together with the attached nitrogen atom to form a piperazine ring substituted with C₁-C₆ alkyl.

Suitable "C₁-C₆ alkylidene" may be methylene, ethylidene, propylidene or butylidene of which the most preferable one is methylene.

Each step for preparing the compound [Ia] is explained in more detail in the following.

### Step a

Suitable salts of the compound [II] may be the same as exemplified for the compound [I].

This reaction is usually carried out in a conventional solvent such as diethyl ether, tetrahydrofuran, dioxane, benzene, acetone, methyl ethyl ketone, methyl isobutyl ketone or any other solvent which does not adversely influence the reaction.

The reaction temperature is not critical and the reaction is usually carried out under cooling or at ambient temperature.

### Step b

Suitable agents which liberate ammonia may be ammonium C₁-C₆ alkanoate[e.g. ammonium formate, ammonium acetate, ammonium propionate and ammonium butyrate], ammonium carbonate, ammonium hydrogencarbonate or ammonium carbamate.

This reaction is usually carried out in a conventional solvent such as water, alcohol [e.g. methanol, ethanol or propanol], dioxane, tetrahydrofuran, methylene chloride, chloroform, diethyl ether, benzene,acetone, methyl ethyl ketone, methyl isobutyl ketone or any other organic solvent which does not adversely influence the reaction, or a mixture thereof.

The reaction temperature is not critical, and the reaction is usually carried out under cooling, at ambient temperature or under warming or heating.

### Step c

The oxidation reaction can be carried out by a conventional method which is applied for the transformation of an N-containing heterocyclic base to an aromatized N-containing heterocyclic compound, for example, by using an oxidizing agent such as manganese dioxide, lead tetraacetate, mercuric acetate, halogen [e.g. iodine or bromine], oxygen,hydrogen peroxide, nickel peroxide, sulfur powder, 2,3-dichloro-5,6-dicyano-1,4-benzoquinone or potassium permanganate.

The present reaction is usually carried out in a conventional solvent such as chloroform, methylene chloride, benzene, toluene, pyridine, ethyl acetate, acetone, methyl ethyl ketone, methyl isobutyl ketone or any other organic solvent which does not adversely influence the reaction.

The reaction temperature is not critical and the reaction is preferably carried out at ambient temperature or under warming to heating.

The obtained compound [Ia] in this step can be separated and isolated from the reaction mixture and purified by methods commonly used for this purpose, for instance, extraction with suitable solvent, column chromatography, reprecipitation or recrystallization.

The compound [Ia] can be prepared more easily and in much better yield by this preparation method without isolation of intermediate products than by the preparation with isolation of intermediate products in each step and than by the preparations described in the above-mentioned EP-A-0228845.

These reaction conditions stated in each step may vary according to the kinds of reactants, solvents and/or other agents.

The following Examples and Reference are given for the purpose of illustrating this invention in more detail.

### Example 1

A solution of N-(2-aminoethyl)morpholine (170.5 g) in methyl isobutyl ketone (200 ml) was added portionwise to a solution of diketene (102 ml) in methyl isobutyl ketone (1.3 ℓ) at -30 to -10°C under stirring and the mixture was stirred at -10 to 0°C for one hour. To the resultant solution containing N-(2-morpholinoethyl)acetoacetamide, was added 3-(3-nitrophenyl)-1-phenyl-2-propen-1-one (220.4 g) and ammonium acetate (100 g) and the mixture was stirred at 80°C for 4.5 hours. The reaction mixture was washed with water and dried over magnesium sulfate. To the filtrate containing 1,4-dihydro-3-(2-morpholinoethylcarbamoyl)-2-methyl-4-(3-nitrophenyl)-6-phenylpyridine was added manganese dioxide (700 g) and the resultant mixture was stirred at 80°C for 2 hours. Manganese dioxide was filtered off and the filtrate was evaporated in vacuo. A mixture of ethyl acetate and water was added to the residue and adjusted to pH 1.0 with 10% hydrochloric acid. The separated aqueous layer was adjusted to pH 9.0 with 20% aqueous potassium carbonate and extracted with a mixture of ethyl acetate and tetrahydrofuran. The extract was washed with brine and dried over magnesium sulfate. The solvent was evaporated in vacuo and the residue was recrystallized from ethanol to give 3-(2-morpholinoethylcarbamoyl)-2-methyl-4-(3-nitrophenyl)-6-phenylpyridine (154 g, yield 39.6%), which was identified as the compound of Example 9-(2) described in EP-A-0228845 and the compound of Reference (3) as mentioned below by their physical data.

### Example 2

The following compounds were obtained according to a similar manner to that of Example 1.
(1) 3-(2-Dimethylaminoethylcarbamoyl)-2-methyl-4-(3-nitrophenyl)-6-phenylpyridine
   This compound was identified as the compound of Example 8 described in EP-A-0228845.
(2) 2-Methyl-4-(3-nitrophenyl)-6-phenyl-3-[2-(4-thiomorpholinyl)ethylcarbamoyl]pyridine and its fumarate
   This compound was identified as the compound of Example 9-(3) described in EP-A-0228845.
(3) 2-Methyl-3-(4-methylpiperazin-1-ylcarbonyl)-4-(3-nitrophenyl)-6-phenylpyridine
   This compound was identified as the compound of Example 9-(1) described in EP-A-0228845.

### Example 3

To a solution of 3-(2-morpholinoethylcarbamoyl)-2-methyl-4-(3-nitrophenyl)-6-phenylpyridine (3 g) in ethanol (60 ml) was added concentrated hydrochloric acid (1.68 ml) and water (3.32 ml). The separated crystal was filtered, washed with ethanol (20 ml) and dried in vacuo to give 3-(2-morpholinoethylcarbamoyl)-2-methyl-4-(3-nitrophenyl)-6-phenylpyridine dihydrochloride (2.91 g).
mp : 269-272°C (dec.)
IR (Nujol) : 3175, 1660 cm⁻¹
NMR (D₂O, δ) : 3.00 (3H, s), 3.1-4.3 (12H, m), 7.6-8.2 (7H, m), 8.35-8.65 (2H, m)

| Elemental analysis : C₂₅H₂₆N₄O₄.2HCl | | | | |
|---|---|---|---|---|
| Calcd. | C 57.81, | H 5.43, | N 10.79, | Cl 13.65 |
| Found | C 57.80, | H 5.47, | N 10.77, | Cl 13.90 |

### Example 4

3-(2-Morpholinoethylcarbamoyl)-2-methyl-4-(3-nitrophenyl)-6-phenylpyridine monohydrochloride (17.09 g) was obtained according to a similar manner to that of Example 3 from 3-(2-morpholinoethylcarbamoyl)-2-methyl-4-(3-nitrophenyl)-6-phenylpyridine (20 g) and a solution of acetyl chloride (3.5 ml) in ethanol (60 ml).
IR (Nujol) : 3300, 3200, 1660, 1650, 1530, 1355 cm⁻¹
NMR (CDCl₃, δ) : 2.66 (3H, s), 2.66-3.5 (6H, m), 3.53-4.20 (6H, m), 7.31-8.76 (9H, m)

### Reference

(1) To a solution of diketene (23.0 ml) in diethyl ether (115 ml) was added dropwise a solution of N-(2-aminoethyl)morpholine (34.7 g) in diethyl ether (300 ml) at -40°C. After stirring for 2 hours at the same condition, the resulting precipitates were collected by filtration and dried in vacuo to give N-(2-morpholinoethyl)acetoacetamide (41.1 g, yield 72.0 %).
   IR (Nujol) : 3290, 1715, 1640 cm⁻¹
   NMR (CDCl₃, δ) : 2.3 (3H, s), 2.4-2.7 (6H, m), 3.2-3.9 (8H, m), 7.3 (1H, s)
(2) Ammonia was bubbled into a solution of N-(2-morpholinoethyl)acetoacetamide (40 g) in a mixture of diethyl ether (400 ml) and tetrahydrofuran (200 ml) at 20°C under water cooling for 3 hours. The resulting precipitates were collected by filtration and dried in vacuo to give N-(2-morpholinoethyl)-3-aminocrotonamide (18.0 g, yield 45.2 %).
   mp : 86-93°C
   IR (Nujol) : 3300, 3120, 1620 cm⁻¹
   NMR (CDCl₃, δ) : 2.7-3.0 (9H, m), 3.6-4.3 (6H, m), 4.8 (1H, s)
(3) A mixture of 3-(3-nitrophenyl)-1-phenyl-2-propen-1-one (20 g) and N-(2-morpholinoethyl)-3-aminocrotonamide (21.9 g) in toluene (200 ml) was refluxed for 55 hours. After allowing to cool at ambient temperature, the reaction mixture was concentrated in vacuo. The residue was subjected to a column chromatography on silica gel eluting with a mixture of ethyl acetate and tetrahydrofuran (100:1 V/V). The fractions containing the object compound were combined and concentrated in vacuo. The residue was recrystallized from ethyl alcohol to give 3-(2-morpholinoethylcarbamoyl)-2-methyl-4-(3-nitrophenyl)-6-phenylpyridine (11.0 g, yield 31.2 %).
   Total yield (1) to (3) : 10.2 %
   mp : 141-142°C
   IR (Nujol) : 3200, 1630, 1565 cm⁻¹

### Example 5

| | |
|---|---|
| 3-(2-Morpholinoethylcarbamoyl)-2-methyl-4-(3-nitrophenyl)-6-phenylpyridine (hereinafter referred to as Compound A) | 100g |
| Hydroxypropylmethyl cellulose | 500 g |
| Lactose | 6.87 kg |
| Low-substituted hydroxypropyl cellulose | 1.5 kg |
| Magnesium stearate | 30 g |

To a suspension of Compound A and hydroxypropylmethyl cellulose in anhydrous ethanol (5 liters) were added lactose and low-substituted hydroxypropyl cellulose, and the resultant mixture was stirred and then the organic solvent was removed under reduced pressure to give solid dispersion composition. After this composition was converted into granules by a conventional method, the granules were further converted with magnesium stearate into tablets by a conventional method, each of which contains 2 mg of Compound A.

### Example 6

| | |
|---|---|
| Compound A | 25.0 g |
| Methyl cellulose | 0.5 g |
| Polyvinylpyrrolidone | 0.05 g |
| Methyl p-hydroxybenzoate | 0.1 g |
| Polysorbate 80 | 0.1 g |
| Lidocain hydrochloride | 0.5 g |
| Distilled water | a proper quantity (total volume 100 ml) |

The above-mentioned ingredients were mixed to give 100 ml of an injection suspension.

## Claims

1. Use of a compound of the formula: 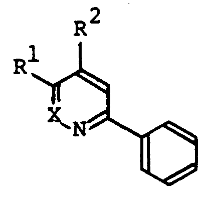 wherein
R¹ is C₁-C₆ alkyl substituted with C₁-C₆ alkyl substituted piperazinyl; carbamoyl substituted with morpholino(C₁-C₆)alkyl, thiomorpholino(C₁-C₆)alkyl
or C₁-C₆ alkylamino(C₁-C₆)alkyl;
piperazinylcarbonyl substituted with C₁-C₆ alkyl;
or ureido substituted with
C₁-C₆ alkylamino(C₁-C₆)alkyl; and
R² is phenyl substituted with nitro, and
X is =N- or 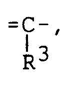 in which R³ is C₁-C₆ alkyl;
or
R² is C₁-C₆ alkyl, and
X is 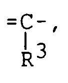 in which R³ is phenyl substituted with nitro;
or its salt for the manufacture of a medicament for the treatment of ischemic disease, depressed cardiac metabolism, reperfusion injury, myocardial infarction, heart failure or angina pectoris in human beings or animals.

2. Use of a compound according to claim 1, wherein R¹ is morpholino(C₁-C₆)alkylcarbamoyl, R² is phenyl substituted with nitro, and X is 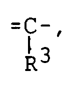 in which R³ is C₁-C₆ alkyl.

3. Use of a compound according to claim 2, which is 3-(2-morpholinoethylcarbamoyl)-2-methyl-4-(3-nitrophenyl)-6-phenylpyridine and its dihydrochloride.

4. Use as claimed in any of claims 1 to 3, wherein the medicament is formulated for oral or parenteral administration.

5. Use as claimed in any of claims 1 to 4, wherein the medicament also comprises one or more organic or inorganic carriers.

## Patentansprüche

1. Verwendung einer Verbindung der Formel 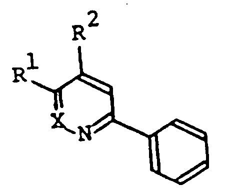 worin bedeuten:
R¹ C₁-C₆-Alkyl, substituiert durch C₁-c₆-Alkyl-substituiertes Piperazinyl; Carbamoyl, substituiert durch Morpholino(C₁-C₆)alkyl, Thiomorpholino(C₁-C₆)alkyl oder C₁-C₆-Alkylamino(C₁-C₆)alkyl; Piperazinylcarbonyl, substituiert durch C₁-C₆-Alkyl; oder Ureido, substituiert durch C₁-C₆-Alkylamino(C₁-C₆)alkyl;
R² Phenyl, substituiert durch Nitro, und
X =N- oder 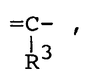 worin R³ für C₁-C₆-Alkyl steht; oder
R² C₁-C₆-Alkyl und
X 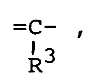 worin R³ für Phenyl, substituiert durch Nitro, steht;
oder ihres Salzes für die Herstellung eines Arzneimittels für die Behandlung von ischämischen Beschwerden (Erkrankungen), eines reduzierten Kardiometabolismus, von Reperfusionsstörungen, eines Myocard-Infarkts, eines Herzversagens oder von Angina pectoris bei Menschen oder Tieren.

2. Verwendung einer Verbindung nach Anspruch 1, worin bedeuten R¹ Morpholino(C₁-C₆)alkylcarbamoyl, R² Phenyl, substituiert durch Nitro, und X 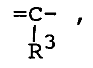 worin R³ für C₁-C₆-Alkyl steht.

3. Verwendung einer Verbindung nach Anspruch 2, bei der es sich handelt um 3-(2-Morpholinoethylcarbamoyl)-2-methyl-4-(3-nitrophenyl)-6-phenylpyridin und sein Dihydrochlorid.

4. Verwendung nach einem der Ansprüche 1 bis 3, bei der das Arzneimittel für die orale oder parenterale Verabreichung formuliert ist.

5. Verwendung nach einem der Ansprüche 1 bis 4, bei der das Arzneimtitel außerdem einen oder mehr organische oder anorganische Träger enthält.

## Revendications

1. Utilisation d'un composé répondant à la formule : 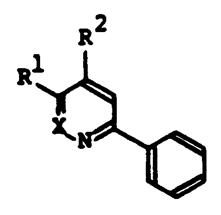 dans laquelle
R¹ est un groupe alkyle en C₁ à C₆ substitué par un groupe pipérazinyle substitué par un groupe alkyle en C₁ à C₆; un groupe carbamoyle substitué par un groupe morpholino(alkyle en C₁ à C₆), thiomorpholino(alkyle en C₁ à C₆) ou (alkyle en C₁ à C₆)amino(alkyle en C₁ à C₆);
pipérazinylcarbonyle substitué par un groupe alkyle en C₁ à C₆;
ou uréido substitué par un groupe (alkyle en C₁ à C₆)amino(alkyle en C₁ à C₆); et
R² est un groupe phényle substitué par un groupe nitro, et
X est =N- ou 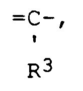 où R³ est un groupe alkyle en C₁ à C₆;
ou
R² est un groupe alkyle en C₁ à C₆, et
X est 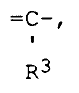 où R³ est un groupe phényle substitué par un groupe nitro;
ou d'un de ses sels pour la fabrication d'un médicament pour le traitement de l'insuffisance coronarienne, de la réduction du métabolisme cardiaque, des lésions de reperfusion, de l'infarctus du myocarde, de l'insuffisance cardiaque ou de l'angine de poitrine, chez l'homme et les animaux.

2. Utilisation d'un composé selon la revendication 1, dans laquelle R¹ est un groupe morpholino(alkyle en C₁ à C₆)carbamoyle, R² est un groupe phényle substitué par un groupe nitro, et X est 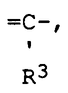 où R³ est un groupe alkyle en C₁ à C₆.

3. Utilisation d'un composé selon la revendication 2, qui est la 3-(2-morpholinoéthylcarbamoyl)-2-méthyl-4-(3-nitrophényl)-6-phénylpyridine et son dichlorhydrate.

4. Utilisation selon l'une quelconque des revendications 1 à 3, dans laquelle le médicament est formulé pour l'administration orale ou parentérale.

5. Utilisation selon l'une quelconque des revendications 1 à 4, dans laquelle le médicament comprend aussi un ou plusieurs supports organiques minéraux.
